(19) Europäisches Patentamt
European Patent Office
Office européen des brevets

(11) EP 1 661 561 A1

(12) EUROPEAN PATENT APPLICATION

(43) Date of publication:
**31.05.2006 Bulletin 2006/22**

(51) Int Cl.:
***A61K 31/09*** *(2006.01)*

(21) Application number: **05291599.8**

(22) Date of filing: **27.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **06.08.2004 FR 0408725**

(71) Applicant: **Takasago International Corporation**
**Tokyo-to 144-8721 (JP)**

(72) Inventors:
- **Warr, Jonathan**
  **75017 Paris (FR)**
- **Kunieda, Satomi**
  **Hiratsuka-shi KANAGAWA-KEN (JP)**

(74) Representative: **Gillard, Marie-Louise et al**
**Cabinet Beau de Loménie**
**158, rue de l'Université**
**75340 Paris Cédex 07 (FR)**

(54) **Cosmetic and dermatological compositions for the treatment of the skin and scalp**

(57) The present invention has as its object a compound of formula (A) :

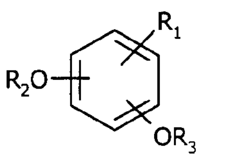

(A)

in which :

- $R_1$ is hydrogen, a hydroxy group, a $C_1$-$C_4$ alkyl group or a $C_1$-$C_3$ alkoxy group,
- $R_2$ and $R_3$, independently of one another, are hydrogen or a $C_1$-$C_4$ alkyl group, preferably a methyl group for use as a blood stimulating agent.

Applications : Cosmetic and dermatological compositions for the traitement of the skin and scalp

EP 1 661 561 A1

## Description

The present invention relates to the treatment of the skin and the scalp.

It has as its object an effective blood stimulating agent which does not give rise to irritation of the epidermis.

It has been known for a long time that capsaicin has the property of dilating blood vessels. However, this compound is highly irritating for the epidermis. It is therefore impossible to use it without giving rise to disorders affecting the epidermis.

Mustard essence and ginger essence are also known as stimulators of blood circulation, but the expected effect is neither satisfactory, nor durable.

Patent application JP 2001-316 317 describes a blood stimulant which is a methoxy-phenolic ether of formula :

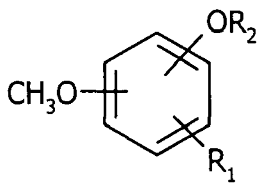

(I)

in which :

- $R_1$ is hydrogen or methoxy ;
- $R_2$ is a $C_1$-$C_{18}$ alkyl or a $C_7$-$C_{24}$ arylalkyl.

A specific family of compounds of formula (I) consists of the dimethoxylated compounds of formula (I) ($R_1$ = methoxy), in which $R_2$ is an alkyl group having at least 4 carbon atoms. In particular, in the described 3,5-dimethoxylated compounds, $R_2$ is a $C_4$ ; $C_6$-$C_{15}$, $C_{17}$ or $C_{18}$ alkyl.

This patent application does not describe dimethoxylated compounds of formula (I) in which $R_2$ is an alkyl group containing 1 to 3 carbon atoms.

The present invention has as its object the compound of formula (A) :

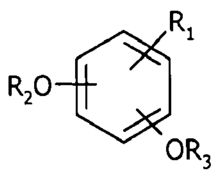

(A)

in which:

- $R_1$ is hydrogen, a hydroxy group, a $C_1$-$C_4$ alkyl group or a $C_1$-$C_3$ alkoxy group,
- $R_2$ and $R_3$, independently of one another, are hydrogen or a $C_1$-$C_4$ alkyl group, preferably a methyl group, for use as a blood stimulating agent.

Particularly preferred compounds according to the invention are compounds of formula (A), in which:

- $R_1$ is a $C_1$-$C_4$ alkyl group, preferably a methyl group or a $C_1$-$C_3$ alkoxy group, preferably a methoxy group and
- $R_2$ and $R_3$ are a methyl group or mixtures thereof.

Among these compounds, 1,3,5-trimethoxybenzene (hereinafter referred to as TMB), 1,3-dimethoxy-5-methylbenzene or mixtures thereof are particularly preferred.

Other compounds preferred with a view to carrying out the invention are the compounds of formula (A) in which $R_1$ is a hydroxy group, $R_2$ and $R_3$ are hydrogen or mixtures thereof.

A particularly preferred compound is 1,3,5-trihydroxybenzene.

Advantageously, there may be used a mixture of one or more methoxylated compounds of formula (A) with one or more hydroxylated compounds of formula (A), in particular mixtures of 1,3,5-trimethoxybenzene and/or 1,3-dimethoxy-5-methylbenzene with 1,3,5-trihydroxybenzene.

The invention is more specifically concerned with the use of at least one compound of above formula (A) as a blood stimulating agent in topical compositions for treatment of the skin and the scalp.

By "topical compositions for treatment of the skin and the scalp" in the present application, it is intended to refer equally to cosmetic compositions, such as creams, lotions, shampoos, after-shampoos, as well as to dermatological compositions for treatment of the skin and the scalp.

The invention also has as its object topical compositions containing at least one compound of formula (A).

Topical compositions are the cosmetic or pharmaceutical compositions as defined hereinabove.

The invention also concerns the use of at least one compound of formula (A) for the preparation of topic compositions useful for stimulating blood circulation.

The compounds of formula (A) are known compounds that are available from commercial sources.

They can be readily manufactured by the methods described or referred to by Liu et al., Organic Preparations and Procedures International, Vol. 35 (2003), 223.

The quantity of compound of formula (A) to be used in the cosmetic or dermatological compositions in order to obtain the blood stimulating effect must be comprised between 0.001 % and 1 % by weight with respect to the total weight of the composition.

The invention will now be described in more detail by the following nonlimiting test.

17 women aged from 20 to 30 years old applied a cream to the whole of the index finger of their left-hand and the blood flow of a part of the median phalanx of the index finger of their left-hand was measured by Doppler (Omega Flow FLO-N1 ; Omega ware Inc.).

The cream used in these tests was a face cream containing the following ingredients:

| Ingredients | Chemical Composition | Marketed by | % by weight |
|---|---|---|---|
| PART A | | | |
| MONTANOV 202 | ARACHIDYL ALCOOL & BEHENYL ALCOOL & ARACHIDYLGLUCOSIDE | SEPPIC | 3.00 |
| LANOL P | GLYCOL PALMITATE | SEPPIC | 5.00 |
| SIMULSOL 165 | POLYETHYLENE GLYCOL STEARATE 100 & GLYCERYL STEARATE | SEPPIC | 2.00 |
| DUB VCI 10 | ISODECYL NEOPENTANOATE | STEARINERIE DUBOIS | 5.00 |
| LANOL 99 | ISONONYL ISONONAOATE | SEPPIC | 9.00 |
| PART B | | | |
| MICROPEARL M305 | CROSS-LINKED POLYMETHYLMETHACRYLATE | SEPPIC | 2.00 |
| SEPITONIC M3 | MAGNESIUM ASPARTATE & ZINC GLUCONATE & COPPER GLUCONATE | SEPPIC | 1.00 |
| SEPICALM S | SODIUM SARCOSINATE & POTASSIUM ASPARTATE & MAGNESIUM ASPARTATE | SEPPIC | 2.00 |
| EDETA BD | SODIUM ETHYLENEDIAMINE TETRAACETATE | BASF | 0.20 |
| WATER | | | 54.80 |
| PART C | | | |
| DC345 | CYLOMETHICONE | LAMBERT RIVIERE | 8.00 |
| SIMULGEL EG | COPOLYMER OF SODIUM ACRYLATE/ ACRYLOYLDIMETHYL TAURATE & ISOHEXADECANE & POLYSORBATE 80 | SEPPIC | 2.50 |
| PART D | | | |

Table continued

| | | | |
|---|---|---|---|
| ALCOHOL 96°C | ETHANOL | | 4.00 |
| SEPICIDE HB | PHENOXYETHANOL/ METHYLPARABEN/ ETHYLPARABEN/PROPYL PARABEN/ BUTYLPARABEN | SEPPIC | 0.50 |

This cream was prepared by first of all mixing the ingredients of part A at 75°C.

The Micropearl M305 was dispersed using magnetic stirring in water at 70-75°C. Subsequently the remainder of the ingredients of part B were mixed with the dispersion of Micropearl M305 and heated until a homogeneous mixture was obtained.

Part A and part B were then mixed and then, while stirring moderately, the silicone DC 345 and the SIMULGEL EG were added one after the other.

Thereafter, ethanol and the preservative SEPICIDE HB, were added whilst stirring at 45-50°C.

The cream obtained was then poured into pots.

This face cream, not containing any perfume, was used for the control experiments (product C1).

The same face cream was also used containing either 1% of rose perfume (product T1), or 0.1% of TMB (product T2).

The rose perfume used to prepare product T1 had the following composition:

| Ingredients | CAS N° | % by weight |
|---|---|---|
| Benzyl alcohol | 100-51-6 | 2.5 |
| Citral | 5392-40-5 | 0.2 |
| Citronellol | 106-22-9 | 7.5 |
| Eugenol | 97-53-0 | 0.4 |
| Geraniol | 106-24-1 | 11 |
| Geranyl acetate | 105-87-3 | 0.3 |
| Cis-3-hexen-1-ol | 928-96-1 | 0.2 |
| Ionone alpha | 127-41-3 | 0.5 |
| Ionone beta | 14901-07-6 | 2.3 |
| Iso cyclo citral | 1335-66-6 | 0.3 |
| Nerol Pure | 106-25-2 | 2.0 |
| Phenylacetaldehyde | 122-78-1 | 1.2 |
| Phenylethyl acetate | 103-45-7 | 6.5 |
| Phenyl ethyl alcohol | 60-12-8 | 62.2 |
| Triethyl citrate | 77-93-0 | 2.85 |

For each cream, the blood circulation was measured before application of the cream, immediately after its application, 30 minutes after application and finally 60 minutes after application.

The cream samples were applied to women on a random basis.

The blood flow before application of the cream was set to 100%.

The results obtained are presented together in the following tables I to III; they show a steady profile of blood flow higher for product T2 with respect to product T1 and for product T2 with respect to control product C1.

No advantage with respect to product C1 appears for product T1.

The results were studied using the signed ranking test of Wilcoxon ["Introduction to Medical Statistics - OUP - M. Bland, ISBN 0192624288"]. This test confirmed a significant difference with a confidence level of 95% for high blood flow with product T2 with respect to product C1 and with respect to product T1, 60 minutes after application of the cream and also a significant difference for higher blood flow for product T2 just after application.

According to this test, for a confidence level of 95%, the absolute differences W have the following meanings:

$$W \leq 34, W \geq 119 \Rightarrow \text{significant difference}$$

$$34 < W < 119 \Rightarrow \text{no significant difference}$$

Table I Blood flow 60 minutes after application of cream. Comparison of C1 with respect to T2.

| Test after 60 minutes | C1 | T2 | difference | absolute difference | ranking |
|---|---|---|---|---|---|
| n° 1 | 127.94 | 171.60 | -43.66 | 43.66 | 12 |
| n° 2 | 117.21 | 59.09 | 58.13 | 58.13 | 9 |
| n° 3 | 121.72 | 28.38 | 93.34 | 93.34 | 6 |
| n° 4 | 684.37 | 163.04 | 521.33 | 521.33 | 1 |
| n° 5 | 62.76 | 97.41 | -34.65 | 34.65 | 13 |
| n° 6 | 2.39 | 13.23 | -10.84 | 10.84 | 16 |
| n° 7 | 44.37 | 107.37 | -62.99 | 62.99 | 8 |
| n° 8 | 33.25 | 102.46 | -69.21 | 69.21 | 7 |
| n° 9 | 52.94 | 76.25 | -23.30 | 23.30 | 15 |
| n° 10 | 56.02 | 100.76 | -44.74 | 44.74 | 11 |
| n° 11 | 67.88 | 222.57 | -154.69 | 154.69 | 4 |
| n° 12 | 50.38 | 101.08 | -50.70 | 50.70 | 10 |
| n° 13 | 98.60 | 126.50 | -27.90 | 27.90 | 14 |
| n° 14 | 184.64 | 177.87 | 6.76 | 6.76 | 17 |
| n° 15 | 36.19 | 264.91 | -228.71 | 228.71 | 2 |
| n° 16 | 41.80 | 166.96 | -125.16 | 125.16 | 5 |
| n° 17 | 115.38 | 309.09 | -193.72 | 193.72 | 3 |
| | | | | Overall ranking W = 33 | |

Table II Blood flow 60 minutes after application of cream. Comparison of T1 with respect to T2.

| Test after 60 minutes | T1 | T2 | difference | absolute difference | ranking |
|---|---|---|---|---|---|
| n° 1 | 113.52 | 171.60 | -58.08 | 58.08 | 10 |
| n° 2 | 90.00 | 59.09 | 30.92 | 30.92 | 13 |
| n° 3 | 19.19 | 28.38 | -9.20 | 9.20 | 15 |
| n° 4 | 161.27 | 163.04 | -1.76 | 1.76 | 17 |
| n° 5 | 35.59 | 97.41 | -61.83 | 61.83 | 9 |
| n° 6 | 105.64 | 13.23 | 92.41 | 92.41 | 4 |
| n° 7 | 181.76 | 107.37 | 74.39 | 74.39 | 7 |
| n° 8 | 67.43 | 102.46 | -35.04 | 35.04 | 11 |
| n° 9 | 57.40 | 76.25 | -18.85 | 18.85 | 14 |
| n° 10 | 28.91 | 100,76 | -71.86 | 71.86 | 8 |

Table continued

| Test after 60 minutes | T1 | T2 | difference | absolute difference | ranking |
|---|---|---|---|---|---|
| n° 11 | 70.36 | 222.57 | -152.21 | 152.21 | 3 |
| n° 12 | 193.48 | 101.08 | 92.40 | 92.40 | 5 |
| n° 13 | 121.42 | 126.50 | -5.08 | 5.08 | 16 |
| n° 14 | 94.65 | 177.87 | -83.23 | 83.23 | 6 |
| n° 15 | 44.99 | 264.91 | -219.92 | 219.92 | 2 |
| n° 16 | 133.81 | 166.96 | -33.15 | 33.15 | 12 |
| n° 17 | 70.43 | 309.09 | -238.66 | 238.66 | 1 |
| | | | | Overall ranking W = 29 | |

Table III Blood flow immediately after application of cream ("after") compared to blood flow 60 minutes after application ("after 60 minutes").

| | after | after 60 min | difference | absolute difference | ranking |
|---|---|---|---|---|---|
| n° 1 | 163.71 | 171.60 | -7.89 | 7.89 | 14 |
| n° 2 | 170.00 | 59.09 | 110.92 | 110.92 | 4 |
| n° 3 | 261.32 | 28.38 | 232.94 | 232.94 | 1 |
| n° 4 | 87.88 | 163.04 | -75.15 | 75.15 | 6 |
| n° 5 | 61.42 | 97.41 | -36.00 | 36.00 | 10 |
| n° 6 | 7.24 | 13.23 | -5.99 | 5.99 | 16 |
| n° 7 | 119.06 | 107.37 | 11.69 | 11.69 | 12 |
| n° 8 | 152.28 | 102.46 | 49.81 | 49.81 | 8 |
| n° 9 | 68.69 | 76.25 | -7.55 | 7.55 | 15 |
| n° 10 | 91.16 | 100.76 | -9.60 | 9.60 | 13 |
| n° 11 | 218.71 | 222.57 | -3.86 | 3.86 | 17 |
| n° 12 | 65.73 | 101.08 | -35.35 | 35.35 | 11 |
| n° 13 | 81.27 | 126.50 | -45.23 | 45.23 | 9 |
| n° 14 | 111.35 | 177.87 | -66.53 | 66.53 | 7 |
| n° 15 | 76.52 | 264.91 | -188.39 | 188.39 | 2 |
| n° 16 | 62.44 | 166.96 | -104.52 | 104.52 | 5 |
| n° 17 | 154.29 | 309.09 | -154.80 | 154.80 | 3 |
| | | | | Overall ranking W = 25 | |

**Claims**

**1.** Compound of formula (A) :

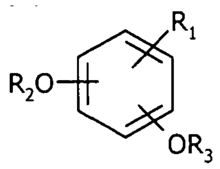

(A)

in which :

- $R_1$ is hydrogen, a hydroxy group, a $C_1$-$C_4$ alkyl group or a $C_1$-$C_3$ alkoxy group,
- $R_2$ and $R_3$, independently of one another, are hydrogen or a $C_1$-$C_4$ alkyl group, preferably a methyl group for use as a blood stimulating agent.

**2.** Compound according to claim 1, **characterised in that** $R_1$ is a $C_1$-$C_4$ alkyl group, $R_2$ and $R_3$ are methyl.

**3.** Compound according to claim 1, **characterised in that** $R_1$ is a $C_1$-$C_3$ alkoxy group, $R_2$ and $R_3$ are methyl.

**4.** Compound according to any of claims 1 to 3, **characterised in that** it is 1,3-dimethoxy-5-methylbenzene or 1,3,5-trimethoxybenzene.

**5.** Compound according to claim 1, **characterised in that** $R_1$ is a hydroxy group, $R_2$ and $R_3$ are hydrogen.

**6.** Compound according to claim 5, **characterised in that** it is 1,3,5-trihydroxybenzene.

**7.** Use of at least one compound of formula (A) :

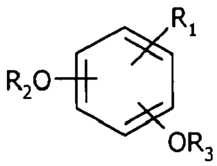

(A)

in which :

- $R_1$ is hydrogen, a hydroxy group, a $C_1$-$C_4$ alkyl group or a $C_1$-$C_3$ alkoxy group,
- $R_2$ and $R_3$, independently of one another, are hydrogen or a $C_1$-$C_4$ alkyl group, preferably a methyl group as a blood stimulating agent in cosmetic compositions for the treatment of the skin and scalp.

**8.** Use according to claim 7, **characterised in that** $R_1$ is a $C_1$-$C_4$ alkyl group, $R_2$ and $R_3$ are methyl.

**9.** Use according to claim 7, **characterised in that** $R_1$ is a $C_1$-$C_3$ alkoxy group, $R_2$ and $R_3$ are methyl.

**10.** Use according to any of claims 7 to 9, **characterised in that** the compound of formula (A) is chosen from 1,3-dimethoxy-5-methylbenzene; 1,3,5-trimethoxybenzene and mixtures thereof.

**11.** Use according to claim 7, **characterised in that** $R_1$ is a hydroxy group, $R_2$ and $R_3$ are hydrogen.

**12.** Use according to claim 11, **characterised in that** the compound of formula (A) is 1,3,5-trihydroxybenzene.

**13.** Use according to any of claims 7 to 12, **characterised in that** the said compound of formula (A) is used in an amount of 0.001 % to 1 % by weight with respect to the total weight of the composition.

**14.** Cosmetic composition for the skin and scalp, **characterised in that** it contains 0.001 % to 1 % of at least one blood stimulating agent of formula (A) as defined in claim 1, with respect to the total weight of the composition.

**15.** Cosmetic composition according to claim 14, **characterised in that** the blood stimulating agent is chosen from 1,3-dimethoxy-5-methylbenzene; 1,3,5-trimethoxybenzene; 1,3,5-trihydroxybenzene and mixtures thereof.

**16.** Use of at least one compound of formula (A) :

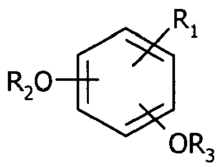

(A)

in which :

- $R_1$ is hydrogen, a hydroxy group, a $C_1$-$C_4$ alkyl group or a $C_1$-$C_3$ alkoxy group,
- $R_2$ and $R_3$, independently of one another, are hydrogen or a $C_1$-$C_4$ alkyl group, preferably a methyl group for the preparation of topical compositions useful for stimulating blood circulation.

**17.** Use according to claim 16, **characterised in that** the compound of formula (A) is used in an amount of 0.001 % to 1 % by weight with respect to the total weight of the said compositions.

**18.** Use according one of claims 16 and 17, **characterised in that** the compound of formula (A) is chosen from 1,3-dimethoxy-5-methylbenzene, 1,3,5-trimethoxybenzene, 1,3,5-trihydroxybenzene and mixtures thereof.

European Patent Office

# EUROPEAN SEARCH REPORT

Application Number
EP 05 29 1599

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,Y | DATABASE WPI<br>Section Ch, Week 200217<br>Derwent Publications Ltd., London, GB;<br>Class B05, AN 2002-126692<br>XP002327164<br>& JP 2001 316317 A (KAO CORP)<br>13 November 2001 (2001-11-13)<br>* abstract * | 1-18 | A61K31/09 |
| X | GB 897 743 A (LOUIS LAFON)<br>30 May 1962 (1962-05-30)<br>* example 4 * | 1 | |
| X | GB 1 000 783 A (ORSYMONDE)<br>11 August 1965 (1965-08-11)<br>* the whole document * | 1,4 | |
| Y | * page 2, left-hand column, lines 35-39 * | 1-18 | |
| X | US 3 093 544 A (LAFON LOUIS)<br>11 June 1963 (1963-06-11)<br>* the whole document * | 1,4,6 | |
| Y | * column 2, line 67 - column 3, line 5 * | 1-18 | TECHNICAL FIELDS SEARCHED (IPC)<br>A61K |
| X | EP 0 988 856 A (SHISEIDO COMPANY, LTD; SHISEIDO COMPANY LIMITED)<br>29 March 2000 (2000-03-29)<br>* paragraphs [0006], [0013] * | 2,3 | |
| A | US 6 268 333 B1 (OKAZAKI YOSHIRO ET AL)<br>31 July 2001 (2001-07-31)<br>* the whole document * | 1-18 | |
| A | EP 0 931 542 A (L'OREAL)<br>28 July 1999 (1999-07-28)<br>* the whole document * | 1-18 | |

The present search report has been drawn up for all claims

1

| Place of search | Date of completion of the search | Examiner |
|---|---|---|
| Munich | 21 March 2006 | Engl, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 29 1599

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2006

| Patent document cited in search report | Publication date | Patent family member(s) | Publication date |
|---|---|---|---|
| JP 2001316317 A | 13-11-2001 | NONE | |
| GB 897743 A | 30-05-1962 | NONE | |
| GB 1000783 A | 11-08-1965 | NONE | |
| US 3093544 A | 11-06-1963 | NONE | |
| EP 0988856 A | 29-03-2000 | CN 1255367 A | 07-06-2000 |
| | | HK 1029049 A1 | 04-02-2005 |
| | | KR 2000023421 A | 25-04-2000 |
| | | TW 550085 B | 01-09-2003 |
| | | US 6315980 B1 | 13-11-2001 |
| US 6268333 B1 | 31-07-2001 | NONE | |
| EP 0931542 A | 28-07-1999 | CA 2255222 A1 | 19-06-1999 |
| | | DE 69821087 D1 | 19-02-2004 |
| | | DE 69821087 T2 | 23-12-2004 |
| | | ES 2214689 T3 | 16-09-2004 |
| | | FR 2772613 A1 | 25-06-1999 |
| | | JP 3162028 B2 | 25-04-2001 |
| | | JP 11246328 A | 14-09-1999 |
| | | US 6054137 A | 25-04-2000 |

EPO FORM P0459

For more details about this annex : see Official Journal of the European Patent Office, No. 12/82